# EUROPEAN PATENT APPLICATION

(11) **EP 1 870 154 A1**
(43) Date of publication of application: **26.12.2007**
(21) Application number: 06012720.6
(22) Date of filing: 21.06.2006
(51) Int. Cl.: B01D 47/06, B01D 53/78, A61L 9/14

(54) **Apparatus for cleaning of room air for odours, dust and microbes**

(71) Applicant: Sermuks, Maris, LV-3708 Auce (LV)
(72) Inventor: Sermuks, Maris, LV-3708 Auce (LV)
(74) Representative: Zvirgzds, Arnolds

(57) **Abstract**

Invention refers to air cleaning systems, which are intended for use in livestock buildings and in different food and other kind of industry, in household insulated rooms for removing odours or aroma and odour-bearing substances, for example, ammonia evaporations, displeasing odour or annoying aroma as well as microbes and dust from room air. The object of the invention is to simplify the air cleaning system, prevent production of big amount of wastewater and on the same time to reduce the total costs. Air cleaning system is provided with recirculation type water supply system maintaining clean water supply to air cleaning chamber (1) connected to used water storage tank (4) which is connected to water purifier (11). Air cleaning chamber (1) is connected to ventilation chamber (2), which is made with several untreated air (A) inlets (3) and to used water storage tank (4) being made as a compact interconnected unit. Water supply system is provided with mist-creating sprayers (5) placed in air cleaning chamber (1) and connected to purified water supply means (6, 10, 12). Exhaust fan (7) is located in zone of outgoing clean air (B). In zone between sprayers (5) and fan (7) in chamber (1) the water-retainer (9) is mounted. Water supply system includes water pump (1) and water purifier (11) as well as clean water reservoir (12), which is connected to additional water supply source. Used water tank (4) is connected to water purifier (11) by discharge pipelines (14). The polluted air (A) is sucked into ventilation chamber (2) and afterwards in air cleaning chamber (1), wherein the sprayers (5) are creating mist removing dust, microbes and odour from air. When the mist is reaching water-retainers (9), discharge water, containing pollutions removed from air, is flowing back to used water tank (4) and afterwards to water purifier (11). Purified water from reservoir (12) through pipeline (6) is discharged back to sprayers (5) creating mist.

## Description

Invention refers to air cleaning systems intended for use in livestock buildings, especially in pigsties, and also in different food and other kind of industries as well as in household isolated rooms in order to clean the air by removing from it the several odours or aroma and odour-bearing substances, for example, ammonia evaporations, displeasing odour or annoying aroma as well as microbes and dust.

Odours or aroma primary is faced in pigsties and henhouses, abattoirs, meet and fish-work industries and food, perfume, pharmaceutical industry and surrounding environment. Multifarious odours or aroma evaporation can be faced every-where. Besides that many of odours has displeasing or allergic impact to working personnel and to people situated close to housing areas so it can harm health and comfort.

In household the odour is neutralised by spraying deodorants, placing air refreshments, which are known, for example, from the description to the Russian Federation patent No. 2085216, ICI⁶ A61L9/01, 1997. Using the deodorants and air refreshments according to the prior art the desired odour inhibition effect is achieved and room is imaginary clean from air pollution although actually air pollution did not disappear.

It is known also air cleaning system, for example, from the description to the Russian Federation patent No 2156924, ICI⁷ F24F3/16, 2000, which is used in household and industrial environment - confectioneries and industrial workshops, in different dining facilities, domestic animals and bird shelters, fish-work industries and elsewhere. Polluted air is removed from room by connecting exhaust fan and discharging it into the environment or, if it is filtered and treated with ozone - back to the room. By performing air exchange in large workshops with powerful air pumps and emission of air with unwanted aroma outside the facilities the surrounding in big area is polluted with unpleasant odour. This odour can become smothery, plaguesome or allergic. Therefore the unpleasant odour is turning the surrounding similar to that which is in livestock buildings and bird shelters, fish-work and other product processing facilities.

The air cleaning system for poultry or pigsty buildings of prior art is known, for example, from Internet web-page publication http://www/bigdutchman.ru (prototype), where is described a system which contains air inlet equipment valve or openings in ceiling as well as forced ventilation equipment for outgoing air. Air cleaning system of prior art comprises air cleaning means located in next - door room, where centralized pumping of outgoing air is effected through large openings in building ending wall. The system includes two or more air cleaning stages, wherein on the first stage the water from the first filtrating wall front side, made of pulp, is dispensed through sprayer group in order to avoid drying out and settlement of dust on filtration wall. On the same time the air humidity increases significantly increasing also absorption capacity of the moistened surface. The humidified air flows through the first filtration wall on which from top - down continuously flows water. The dust is discharged down to the first water reservoir. Since the dust usually contains ammonia and unpleasant odours therefore the significant part of these substances is separated from air already on this stage. Other particles precipitate in reservoir, which is emptied within certain time periods, but the sediment is used for agricultural purposes. On the next stage the air flows through the second filtration wall, which mainly serves for air cleaning from ammonia, small dust particles and unpleasant odour. Between the first and second filtration walls the control corridor is located. By adding sulphuric acid the air cleaning quality to some extent increases. As result of chemical reaction ammonia transforms to sulphate. The water afterwards is discharged as wastewater. If the requirements regarding unpleasant odour removing are especially high, the third - biological air filtration stage can be applied. For this purpose the bio-filter consisting of wood chips, earth, peat and the like is used in order to transform the odour micro-biologically. For system operation the control computer is used, which provides operation safety as well as maintenance of the entire system. System operation indicators such as indicators of water pH level, pressure differences in separate filtration stages, air temperature and humidity, power and water consumption are controlled using display. Disadvantages of the air cleaning system of the prior art concern its complexity; operation produces big amount of wastewater, which contains environmentally unfriendly substances; also operation costs are high.

The present invention has for its object to simplify the air cleaning system, prevent production of big amount of wastewater and on the same time to reduce the total costs.

In conformity of invention there is provided a system for cleaning the room air by removing the odours, odour-bearing substances, dust and microbes from the air, occurring in manufacturing, working or animal breeding process in isolated rooms, including polluted air intake, outlet for the clean outgoing air, ventilation means, at least one set of air cleaning means, means for establishing water flow, means for used water discharge and control equipment. According to the invention the air cleaning system is characterised in that it is provided with a water supply system of recirculation type maintaining clean water supply to air cleaning means in the form of chamber connected to used water storage tank, which is connected to water purifier. Thus the air cleaning chamber, ventilation chamber/ or chambers and used water storage tank is made as a compact interconnected unit. Water supply system is provided with mist-creating sprayers located in air cleaning chamber and connected to purified water supply means; but in the said chamber a water-retainer for preventing escape of mist from the room is installed; wherein a water supply system includes a water recirculation pump and a reservoir for additional water supply. In embodiment with vertical type of air cleaning chamber an exhaust fan is installed in the used air zone, but in embodiment with horizontal type of air cleaning chamber - in zone of polluted air intake; besides that in embodiment with horizontal type of chamber the mist-creating sprayers are located in zone between at least of two water-retainers.

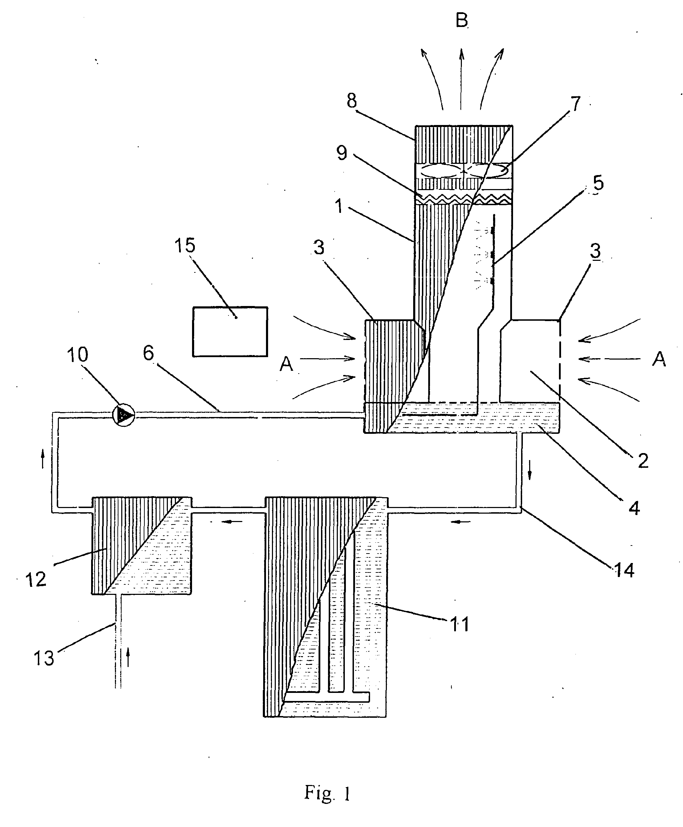

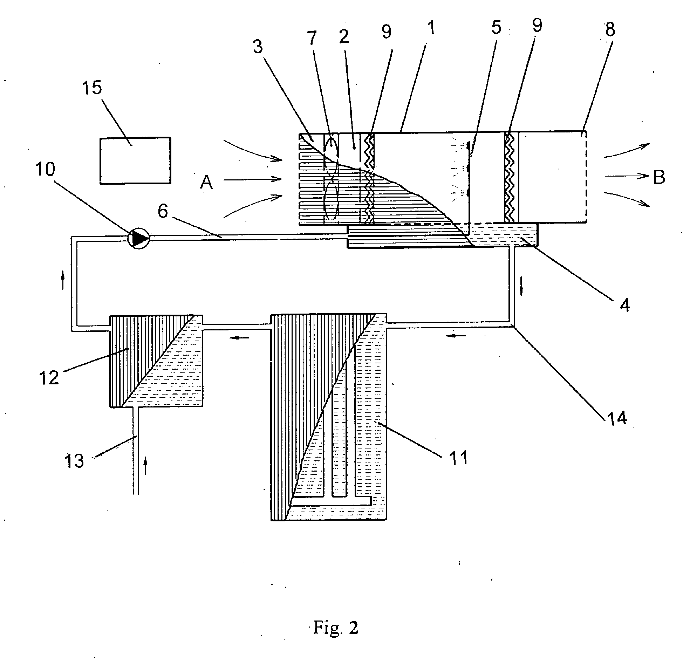

The invention is illustrated by the accompanying drawings, where on Fig. 1 is shown the principal diagram of implementation of the system for cleaning of air with vertical type air cleaning chamber, on Fig. 2 - the principal diagram of the air cleaning system with horizontal type of air cleaning chamber.

A system implemented according to the present invention for cleaning of room air by removing odours, odour-bearing substances, dust and microbes of the vertical type contains an air cleaning chamber 1 (Fig. 1) connected to ventilation chamber 2, which is provided with several inlets 3 for polluted ingoing air A. Air cleaning chamber 1 is connected to an used water storage tank 4. Thus air cleaning chamber 1, ventilation chamber or chambers 2 and used water storage tank 4 is actually made as one compact unit of air cleaning system. In air cleaning chamber 1 there are located several mist-creating sprayers 5, connected to clean water supply through pipeline 6. Exhaust fan 7 is located in outlet zone 8 of the outgoing clean air B. Between sprayers 5 and exhaust fan 7 in air cleaning chamber 1 a water-retainer 9 is mounted. Air cleaning system is provided with recirculation type water supply system, which includes the mentioned used water storage tank 4, a water pump 10 and water purifier 11 as well as a clean water reservoir 12, which by means of pipeline 13 is connected to additional water supply source (not shown). Used water storage tank 4 is connected to water purifier 11 through discharge pipeline 14. The automatic device with panel board 15 is intended for control of air cleaning system operation.

In isolated rooms with doors, windows and ventilation openings, which can be rooms in pigsties, cattle farm, abattoir, meet, fish-fork and food production or rooms in any other kind of industry one or more proposed air cleaning units can be installed by fixing them to ventilation openings.

Air cleaning system is operating as follows. In ventilation chamber 2 the polluted air A containing dust, microbes, solid-state odour-bearing particles and unpleasant or undesirable odours, including ammonia evaporations, is sucked in ventilation chambers 2 through inlets 3. In vertical air cleaning chamber 1 the sprayers 5 are creating mist. When the ingoing polluted air A is passing through the mist zone, the water is separating dust, microbes, solid-state particles and ammonia bearing-substances, which are source of undesired odour. Chamber 1 is provided also with a water-retainer 9 mounted before the exhaust fan 7. Water-retainer 9 prevents escape of the water outside the isolated room. Water from retainer 9 is flowing back through ventilation chamber 2 into the used water storage tank 4. Water supply system, which includes the said used water storage tank 4 is operating in recirculation regime. Since the used water in tank 4 has absorbed unpleasant odour and dust, in order to not saturate it with not necessary pollution, it is discharged through discharge pipeline 14 to water purifier 11, which is operating on basis of aeration principle. After purification the water flows to clean water reservoir 12 from where it is supplied back through pipeline 6 to the system for cleaning of air. In order to ensure certain amount of water circulating in system, reservoir 12 by means of pipeline 13 is connected to additional clean water supply source (not shown). In order to ensure the water discharge to water purifier 11, purified water supply to sprayers 5 creating mist in air cleaning chamber 1 and water back flow to storage tank 4 and control the operation of the system, any known automatic operation device 15 with panel board can be used.

In drawing Fig. 2 is shown modified, i.e., horizontal type air cleaning system, wherein air cleaning chamber 1 is located horizontally, but sprayers 5 creating mist are placed in air cleaning chamber 1 between two water-retainers 9. The exhaust fan 7 is placed in inlet zone 3 of ingoing polluted air A of air cleaning chamber 1.

Vertical or horizontal type air cleaners can be used depending on place of application and type; they differ only by specific structural features.

If necessary, the air cleaning system can contain additional stages with any other filtration elements depending on air odour specification.

The cleaned air can be returned back to the rooms, as result of which in cold periods of seasons the heat energy can be saved, which significantly reduces costs.

Proposed air cleaning system is characterised by:
- compactness and high degree of efficiency;
- economical operation and low maintenance costs;
- easy maintenance.

The proposed air cleaning system can be manufactured and used in any branch of industry and economy, everywhere where it is necessary to remove odour from air, which is going out from the rooms and to improve air quality in environment.

## Claims

1. System for cleaning of room air by removing odour, odour-bearing substances, dust and microbes from it, which includes polluted air intake, outlet of purified air, ventilation means, at least one set of air cleaning means, means for establishing water flow, means for used water discharge and control equipment, **characterized in that** it is provided with recirculation type water supply system maintaining clean water supply to air cleaning chamber (1) connected to used water storage tank (4) which is connected to water purifier (11).

2. System for cleaning of room air according to claim 1, **characterized in that** the air cleaning chamber (1), ventilation chamber or chambers (2) and used water storage tank (4) is made as a compact interconnected unit.

3. System for cleaning of room air according to claim 1 or 2, **characterized in that** the water supply system is provided with mist-creating sprayers (5) placed in air cleaning chamber (1) and connected to purified water supply means (6, 10, 12).

4. System for cleaning of room air according to any of preceding claims, wherein a water-retainer (9) for preventing escape of mist from the room is installed in air cleaning chamber (1).

5. System for cleaning of room air according to any of preceding claims, wherein the water supply system includes water recirculation pump (10) and reservoir (12) for additional clean water supply.

6. System for cleaning of room air according to any of preceding claims, wherein an exhaust fan (7) in air cleaning chamber (1) of vertical type is installed in the outgoing clean air zone (8).

7. System for cleaning of room air according to any of preceding claims wherein an exhaust fan (7) in air cleaning chamber (1) of horizontal type is installed in inlet zone (3) of ingoing polluted air.

8. System for cleaning of room air according to claim 6 wherein mist-creating sprayers (5) are placed in air cleaning chamber (1) between two water-retainers (9).
